# FASCICULE DE BREVET EUROPEEN

(11) **EP 1 756 266 B1**
(45) Date de publication et mention de la délivrance du brevet: **19.11.2008**
(21) Numéro de dépôt: 05757117.6
(22) Date de dépôt: 18.04.2005
(51) Int. Cl.: C12N 5/02, A01N 1/02

(54) **MILIEU SYNTHETIQUE DE CULTURE ET/OU DE CONGELATION ET/OU DE CONSERVATION ET/OU DE TRANSFERT D'EMBRYONS OU DE CELLULES**
SYNTHETISCHES MEDIUM ZUM KULTIVIEREN UND/ODER ZUM EINFRIEREN UND/ODER KONSERVIEREN UND/ODER TRANSFERIEREN VON EMBRYONEN ODER ZELLEN
SYNTHETIC MEDIUM FOR THE CULTURE AND/OR FREEZING AND/OR PRESERVATION AND/OR TRANSFER OF EMBRYOS OR CELLS

(30) Priorité: 20.04.2004 FR 0404154
(43) Date de publication de la demande: 28.02.2007
(73) Titulaire: Ecole Nationale Vétérinaire de Nantes, 44307 Nantes Cedex 03 (FR)
(72) Inventeur: TAINTURIER, Daniel, F-44470 Carquefou (FR); MARTAL, Jacques, F-78350 Jouy-en-Josas (FR); NEIRA, Jorge Alberto, c/o Mr. et Mme CHENE, F-29760 Audierne (FR)
(74) Mandataire: Laget, Jean-Loup
(86) Numéro de dépôt international: PCT/FR2005/000925
(87) Numéro de publication internationale: WO 2005/108555

(56) Documents cités:
- EP-A- 1 176 190
- WO-A-92/01040
- WO-A-99/67364
- US-A- 6 150 163
- HOLM P ET AL: "High bovine blastocyst development in a static in vitro production system using SOFaa medium supplemented with sodium citrate and myo-inositol with or without serum-proteins" THERIOGENOLOGY, vol. 52, no. 4, septembre 1999 (1999-09), pages 683-700, XP002223348 ISSN: 0093-691X cité dans la demande
- TERVIT H R ET AL: "Successful culture in vitro of sheep and cattle ova." JOURNAL OF REPRODUCTION AND FERTILITY, vol. 30, no. 3, septembre 1972 (1972-09), pages 493-497, XP000117968 ISSN: 0022-4251 cité dans la demande

## Description

La présente invention concerne un milieu de culture et/ou de congélation et/ou de conservation et/ou de transfert d'embryons ou de cellules, d'origine humaine ou animale, ce milieu se présentant sous forme d'une solution comprenant un milieu de base exempt de protéines naturelles d'origine animale ou humaine, les constituants de ce milieu de base incluant des sels essentiellement de calcium, de sodium et de potassium, au moins un acide aminé ou un dérivé d'acide aminé, de préférence des vitamines, et éventuellement au moins un antibiotique.

De nombreux milieux de culture permettent aujourd'hui un développement favorable des cellules cultivées en présence de produits biologiques, en particulier de protéines naturelles d'origine animale ou humaine, ajoutés au milieu de culture. A titre de produits biologiques constitués de protéines naturelles d'origine animale ou humaine largement utilisés aujourd'hui dans les milieux de culture, on trouve en particulier le sérum de veau foetal, i'aibumine de sérum bovin ou humain, le sérum de vache en oestrus, etc. Ainsi, par exemple, actuellement le milieu le plus souvent utilisé pour la culture embryonnaire in vitro est le SOF (SOF Tervit et al. 1972), un milieu chimiquement défini ou semi-défini à partir de sécrétion d'oviducte de synthèse, supplémenté en acides aminés essentiels et non essentiels, pyruvate, lactate, myo-Inositol, glutamine et citrate en présence de sérum de veau foetal (Holm et al 1999). Les cultures sont réalisées sous une faible pression en oxygène afin de limiter les réactions d'oxydation. Toutefois, la présence de sérum de veau foetal engendre un certain nombre d'inconvénients. En effet, les produits biologiques, tels que le sérum de veau foetal, présentent de grandes variations quant à la composition de leurs constituants et peuvent incidemment être une source éventuelle de transmission de virus ou d'autres agents pathogènes et même provoquer des altérations métaboliques au cours du développement embryonnaire foetal. En outre, le sérum de veau foetal présente de nombreuses inclusions lipidiques qui rendent par exemple plus difficile la congélation.

On est donc aujourd'hui à la recherche, pour toutes les cellules ou embryons dont le développement ne peut s'effectuer en l'absence de telles protéines naturelles d'origine animale ou humaine, de milieux dont la composition pourrait être entièrement synthétique pour écarter tous les inconvénients mentionnés ci-dessus.

Dans la demande internationale WO 92/01040, il est décrit l'utilisation d'un milieu SOF complémenté en LIF.

Le brevet EP-A-1.176.190 décrit quant à lui un milieu de culture contenant au moins deux facteurs de croissance et un corticoïde. Parmi les milieux de croissance décrits, il n'est jamais mentionné le TGF β1 (Transforming growth factor β1), ni le bFGF (fibroblast growth factor basique) ni le LIF.

La demande internationale WO 99/67364 décrit l'influence du GM-CSF (Granulocyte macrophage colony stimulating factor) sur la culture in vitro d'embryons.

Le document US-A-6.150.163 montre, en particulier dans son exemple 10, l'effet bénéfique d'une association TGF β1 (Transforming growth factor β1) + IGF-1 (Insulin-like growth factor I). Toutefois, dans ce cas, ces deux facteurs de croissance sont utilisés dans un milieu contenant de l'albumine de sérum humain.

En conséquence, il ne peut être considéré que cette association de facteurs de croissance, bien que mentionnée, permette d'en déduire qu'elle peut être utilisée en substitution de protéines d'origine naturelle, humaine ou animale. Au contraire, la présence d'albumine tend à suggérer le contraire à l'homme du métier.

Un but de la présente invention est donc de proposer un milieu de culture et/ou de congélation et/ou de conservation et/ou de transfert de cellules ou d'embryons, d'origine humaine ou animale, dont la conception permet la réalisation d'un milieu synthétique exempt de protéines naturelles d'origine animale ou humaine.

A cet effet, l'invention a pour objet un milieu synthétique de culture et/ou de congélation et/ou de conservation et/ou de transfert d'embryons ou de cellules d'origine humaine ou animale, ce milieu se présentant sous forme d'une solution comprenant un milieu de base exempt de protéines naturelles d'origine animale ou humaine, les constituants de base de ce milieu de base incluant des sels essentiellement de calcium, de sodium et de potassium, au moins un acide aminé ou un dérivé d'acide aminé, de préférence des vitamines, et éventuellement au moins un antibiotique, caractérisé en ce que ledit milieu contient, en sus du milieu de base, au moins un succédané aux protéines naturelles d'origine animale ou humaine, ce succédané à base de molécules recombinantes comprenant au moins la combinaison du TGF β1 (Transforming growth factor β1) et de l'IGF-I (Insulin-like growth factor I), avec au moins un facteur de croissance ou cytokine choisi dans le groupe constitué par ie LiF (Leukemia inhibitory factor), le GM-CSF (Granulocyte macrophage colony stimulating factor) et le bFGF (fibroblast growth factor basique).

L'invention a encore pour objet une utilisation de la combinaison du TGF β1 recombinant, de l'IGF-I recombinant et d'au moins un facteur de croissance ou cytokine recombinant choisi dans le groupe constitué par le LIF, le GM-CSF et le bFGF en tant que substitut de protéines naturelles d'origine animale ou humaines telles que le sérum de veau foetal dans la fabrication de milieu de culture et/ou de congélation et/ou de conservation et/ou de transfert d'embryons ou de cellules, d'origine humaine ou animale.

L'invention sera bien comprise à la lecture de la description suivante d'exemples de réalisation, en référence aux dessins annexés dans lesquels :
la figure 1 représente, sous forme d'un tableau comparatif, les résultats exprimés en termes de blastocystes éclos et de blastocystes totaux après 9 jours de culture dans des milieux de composition différente ;
la figure 2 représente, sous forme de graphique, l'effet de l'association IGF-1, TGF β1, GM-CSF, bFGF et LIF sur le temps de culture et le rendement en blastocystes entre le sixième et le neuvième jour après la fécondation ;
la figure 3 représente, sous forme de graphique, le nombre de cellules de la masse cellulaire interne (MCI) et du trophoblaste (tropho) des blastocystes produits in vitro et cultivés jusqu'au neuvième jour en présence d'IGF-I, TGF β1, GM-CSF, bFGF et LIF;
la figure 4 représente, sous forme d'un tableau, l'incidence du TGF β1 recombinant en présence ou absence de SVF (sérum de veau foetal), sur le développement d'embryons produits in vitro et cultivés jusqu'au neuvième jour après ia fécondation ;
la figure 5 représente, sous forme d'un tableau, l'incidence de l'IGF-I recombinant en présence ou absence de SVF (sérum de veau foetal), sur le développement d'embryons produits in vitro et cultivés jusqu'au neuvième jour après la fécondation ;
la figure 6 représente, sous forme d'un tableau, l'incidence du LIF recombinant en présence ou absence de SVF (sérum de veau foetal), sur le développement d'embryons produits in vitro et cultivés jusqu'au neuvième jour après la fécondation ;
la figure 7 représente, sous forme d'un tableau, l'incidence du GM-CSF recombinant en présence de SVF (sérum de veau foetal), sur le développement d'embryons produits in vitro et cultivés jusqu'au neuvième jour après la fécondation et
la figure 8 représente, sous forme d'un tableau, l'incidence du bFGF recombinant en présence ou absence de SVF (sérum de veau foetal), sur le développement d'embryons produits in vitro et cultivés jusqu'au neuvième jour après la fécondation.

Comme mentionné ci-dessus, le milieu de culture et/ou de congélation et/ou de conservation et/ou de transfert d'embryons ou de cellules, objet de l'invention, est plus particulièrement destiné à être appliqué à des embryons ou à des cellules, d'origine humaine ou animale. Dans ce qui suit, ce milieu de culture sera plus particulièrement décrit dans le cas d'une application à la culture de zygotes de bovins en vue de l'obtention de blastocystes. Toutefois, toute autre application à des cellules ou embryons nécessitant jusqu'à aujourd'hui la présence de protéines naturelles d'origine animale ou humaine dans le milieu de culture pour leur développement peut être envisagée de manière équivalente.

Ce milieu se présente sous forme d'une solution comprenant un milieu de base exempt de protéines naturelles d'origine animale ou humaine. Ce milieu de base inclut des sels essentiellement de calcium, de sodium et de potassium, au moins un acide aminé ou un dérivé d'acide aminé, de préférence des vitamines, et éventuellement au moins un antibiotique. Ainsi, ce milieu de base, qui sera par la suite appelé SOF, peut être obtenu par mélange d'au moins cinq solutions, la première solution présentant, pour un litre de solution aqueuse, la composition suivante :

| | |
|---|---|
| NaCl (g) | 40 - 80 g/L |
| KH₂PO₄ (g) | 0,5 - 2,5 g/L |
| KCl | 4 - 6 g/L |
| MgSO₄7H₂O | 1 - 3 g/L |
| Na - Lactate (mL) | 5 - 7 mL/L |
| H₂O | |

la deuxième solution présentant, pour un litre de solution aqueuse, la composition suivante :

| | |
|---|---|
| NaHCO₃(g) | 15 - 30 g/L |
| Rouge de phénol | 0,05 - 0,2 g/L |

la troisième solution présentant pour un litre de solution aqueuse, la composition suivante :

| | |
|---|---|
| Na - pyruvate (g) | 6 - 10 g/L |

la quatrième solution présentant pour un litre de solution aqueuse, la composition suivante :

| | |
|---|---|
| CaCl₂2H₂O(g) | 20 - 30 g/L |

la cinquième solution, correspondant au milieu de base, présentant la composition telle que suit :

| | |
|---|---|
| H₂O (g) | |
| Citrate de sodium, tribasique (g) | 0,05 - 0,2 g/L |
| Myo-inositol (g) | 0,4 - 0,6 g/L |
| Première solution (mL) | 10 % en volume |
| Deuxième solution (mL) | 10 % en volume |
| Troisième solution (mL) | 1 % en volume |
| Quatrième solution (mL) | 1 % en volume |
| BME 50x (mL) | 20 - 40 mL/L |
| MEM 100x (mL) | 5 - 20 mL/L |
| Glutamine 200 mM (mL) | 0,5 - 2 mUL |
| Gentamycine (mL) | 4 - 6 mL/L |

le milieu BME ayant une composition exprimée en g/L telle que suit :

| | |
|---|---|
| L-Arginine-HCl | 1,05 g/L |
| L-Cystine | 0,6 g/L |
| L-Histidine | 0,4 g/L |
| L-Isoleucine | 1,3 g/L |
| L-Leucine | 1,3 g/L |
| L-Lysine-HCl | 1,849 g/L |
| L-Methionine | 0,375 g/L |
| L-Phénylalanine | 0,825 g/L |
| L-Threonine | 1,2 g/L |
| L-Tryptophane | 0,2 g/L |
| L-Tyrosine | 0,9 g/L |
| L-Valine | 1,175 g/L |

tandis que le milieu MEM 100x a une composition exprimée en g/L telle que suit :

| | |
|---|---|
| L-Alanine | 0,89 g/L |
| L-Asparagine-H₂O | 1,5 g/L |
| Acide L-Aspartique | 1,33 g/L |
| Acide L-Glutamique | 1,47 g/L |
| Glycine | 0,75 g/L |
| L-Proline | 1,15 g/L |
| L-Sérine | 1,05 g/L |

Ce milieu contient, en sus du milieu de base, au moins un succédané aux protéines naturelles d'origine animale ou humaine. Ce succédané formé au moins par la combinaison du TGF β1 (Transforming growth factor B) et de l'IGF-I (insulin-like growth factor I), avec au moins un facteur de croissance ou cytokine choisi dans le groupe constitué par le LIF (Leukemia inhibitory factor), le GM-CSF (Granulocyte macrophage colony stimulating factor) et le bFGF (fibroblast growth factor basique), tous les facteurs de croissance ou cytokines mentionnés ci-dessus étant présents dans le milieu sous forme de molécules recombinantes.

Dans un premier mode de réalisation de l'invention, le milieu comprend un succédané aux protéines naturelles d'origine animale ou humaine comprenant au moins par la combinaison du TGF β1, de l'IGF-I et du LIF présentes sous forme de molécules recombinantes.

Dans un second mode de réalisation préféré de l'invention, le succédané comprend au moins la combinaison du TGF β1 et de l'IGF-I avec au moins deux facteurs de croissance ou cytokine choisis dans le groupe constitué par le LIF, le GM-CSF et le bFGF présentes sous forme de molécules recombinantes.

Le succédané aux protéines naturelles d'origine animale ou humaine peut, dans un mode de réalisation particulier de l'invention, comprendre au moins la combinaison de molécules recombinantes comprenant le TGF β1, l'IGF-I, le LIF, le GM-CSF, le bFGF et l'IGF-II.

Dans ce succédané, les facteurs de croissance ou cytokine recombinants sont présents chacun dans le milieu à une concentration comprise dans la plage [10 - 100] ng/mL, de préférence voisine de 50 ng/mL. Il peut être en outre prévu, en fonction de la destination de cryopréservation d'embryons ou de cellules, d'ajouter à ce milieu de culture un agent cryoprotecteur choisi de préférence dans le groupe constitué par l'éthylène glycol 0,15 M, le glycérol 0,1 M et le propylène glycol 0,15 M.

La combinaison de l'IGF-I, de TGF β1, du GM-CSF, du bFGF et de LIF à 50 ng/mL dans un milieu de base SOF, tel que décrit ci-dessus, montre un effet bénéfique sur le développement des embryons produits in vitro et cultivés jusqu'au neuvième jour après la fécondation. Grâce à cette combinaison, des résultats similaires à ceux obtenus en présence de sérum de veau foetal (SVF) à une concentration de 10 % sont observés. Ce milieu, constitué de SOF combiné avec de l'IGF-I, TGF β1, GM-CSF, bFGF et LIF à 50 ng/mL sera appelé MI pour milieu de l'invention dans ce qui suit. Ce milieu MI contient en outre de l'IGF-II dont la présence s'avère non déterminante pour l'obtention d'un effet équivalent à celui obtenu en présence de protéines naturelles d'origine animale ou humaine.

Pour montrer cet effet bénéfique, trois protocoles ont été testés. Le premier protocole consiste à utiliser un milieu de culture MI, tel que mentionné ci-dessus. Le deuxième protocole consiste à utiliser un milieu de culture comprenant le milieu de base SOF+ 10 % de sérum de veau foetal (SVF). Le troisième protocole consiste à utiliser le milieu de base SOF seul en tant que témoin. Les résultats obtenus ont montré que l'utilisation du milieu de culture MI avec sa combinaison de cytokines et de facteurs de croissance, tels que décrits ci-dessus permet d'obtenir, en terme de rendement en blastocystes, en particulier en blastocystes éclos, des résultats équivalents, voire supérieurs à l'utilisation d'un milieu de base supplémenté avec 10 % de sérum de veau foetal. Le rendement est significativement supérieur au rendement obtenu avec l'utilisation du milieu de base SOF seul.

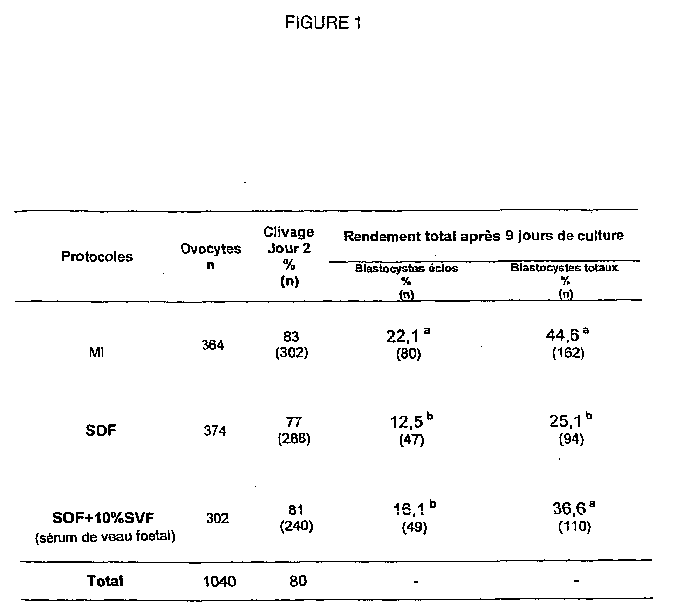

On constate par ailleurs, comme l'illustre la figure 1, que le rendement en blastocystes éclos après 9 jours de culture dans le cadre du protocole MI est significativement supérieur aux deux autres protocoles. Ces résultats montrent que la combinaison de cytokine et de facteurs de croissance avec le milieu de base SOF améliore clairement le potentiel de développement des embryons.

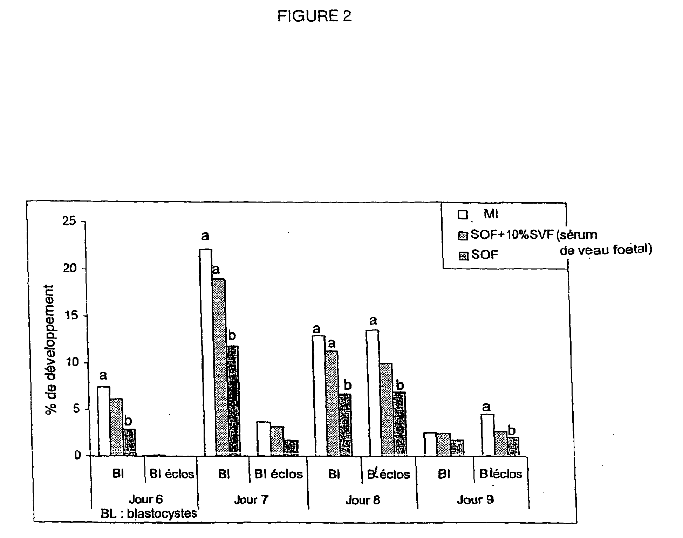

Comme l'illustre la figure 2, les résultats montrent qu'à J 6, J 7 et J 8, le protocole améliore la production de blastocystes par rapport aux deux autres protocoles, ce qui explique son résultat significativement supérieur sur le nombre de blastocystes totaux à J 9. Quant à la proportion des blastocystes éclos, particulièrement à J 8, le protocole MI donne de meilleurs résultats par rapport aux deux autres protocoles.

Par ailleurs, les effets de ce milieu de culture sur la qualité de l'embryon produit ont également été testés. Pour ce faire, il est procédé à un comptage des cellules de la masse cellulaire interne (CMI) et du trophoblaste (TROPHO) des embryons produits in vitro et cultivés dans le milieu. A nouveau, trois protocoles ont été suivis, à savoir un premier protocole utilisant le milieu MI, un deuxième protocole utilisant le milieu de base SOF supplémenté avec 10 % de sérum de veau foetal (SVF) et un troisième protocole utilisant le milieu de base (SOF) seul.

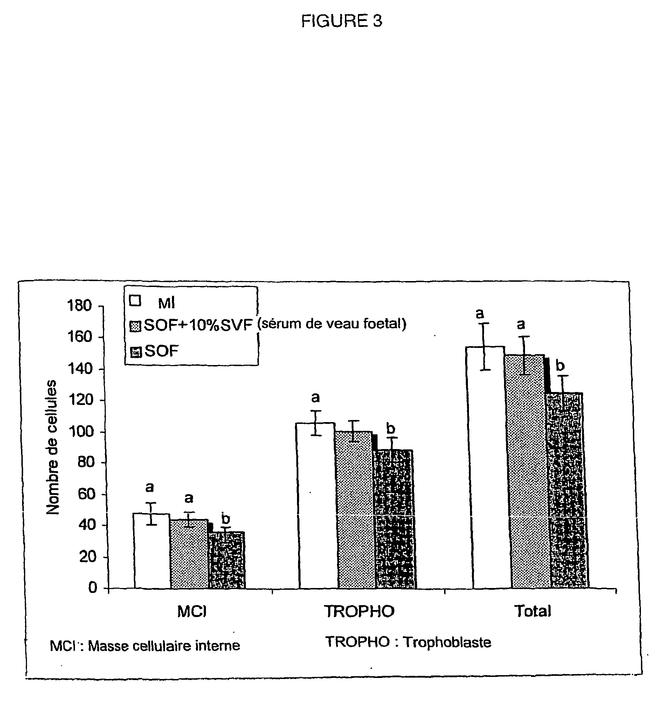

Les résultats obtenus et représentés à la figure 3 montrent que les embryons cultivés dans les milieux MI et milieu de base + 10 % de sérum de veau foetal ont significativement plus de cellules que les embryons cultivés en milieu de base SOF seul.

Par ailleurs, le milieu MI, objet de l'invention, semble fournir de meilleurs résultats que le milieu de base supplémenté avec 10 % de sérum de veau foetal. En conclusion, les résultats obtenus montrent que le milieu synthétique MI décrit ci-dessus, constitué du milieu de base SOF et des facteurs de croissance et des cytokines recombinant IGF-1, TGF β1, GM-CSF, bFGF et LIF améliore clairement le potentiel de développement des embryons produits in vitro par rapport au milieu de base SOF. La présence de ces facteurs de croissance et cytokines astucieusement combinés dans le milieu de culture de base SOF favorise l'augmentation du nombre de cellules de la masse cellulaire interne et du trophoblaste et en conséquence améliore le taux d'éclosion des blastocystes. Les résultats obtenus montrent que la combinaison des facteurs de croissance et des cytokines décrite ci-dessus peut être utilisée en tant que substitut du sérum de veau foetal en fournissant des résultats similaires voire meilleurs.

Il doit être noté que des résultats similaires mais légèrement moins bons pourraient être obtenus avec un milieu MI₂ contenant du SOF, TGF β1, IGF-1 et du LIF.

Les mêmes conclusions s'appliquent pour un milieu MI 3 comprenant du SOF, TGF β1, IGF-1, bFGF ou pour un milieu MI 4 contenant du SOF, TGF β1, IGF I, GM-CSF.

A l'inverse, on constate que chaque cytokine ou facteur de croissance pris isolément ne permet pas d'obtenir un effet équivalent au sérum de veau foetal (SVF) à une concentration de 5 à 10 %.

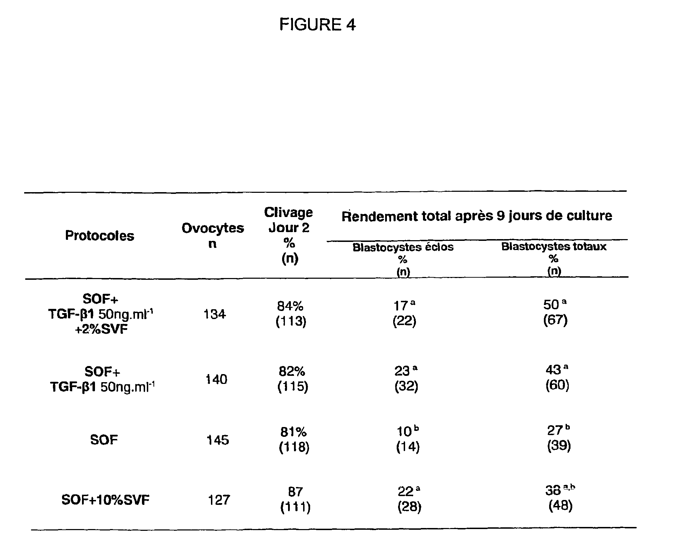

Ainsi, le tableau représenté à la figure 4 montre l'incidence du TGF β1 recombinant en présence ou en absence de SVF (sérum de veau foetal), sur le développement d'embryons produits in vitro et cultivés jusqu'au neuvième jour après la fécondation.

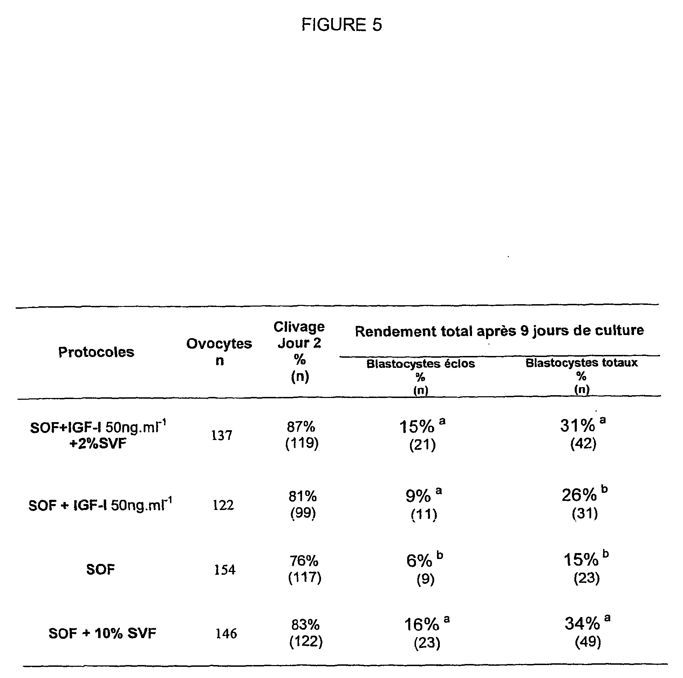

Le tableau représenté à la figure 5 représente l'incidence de l'IGF-I recombinant en présence ou en absence de SVF (sérum de veau foetal), sur le développement d'embryons produits in vitro et cultivés jusqu'au neuvième jour après la fécondation.

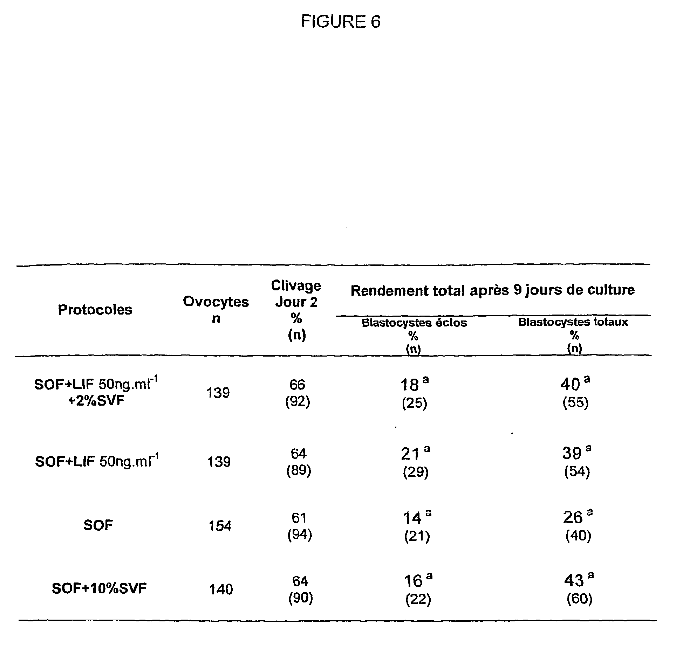

Le tableau représenté à la figure 6 illustre quant à lui l'incidence du LIF recombinant en présence ou en absence de SVF (sérum de veau foetal), sur le développement d'embryons produits in vitro et cultivés jusqu'au neuvième jour après la fécondation.

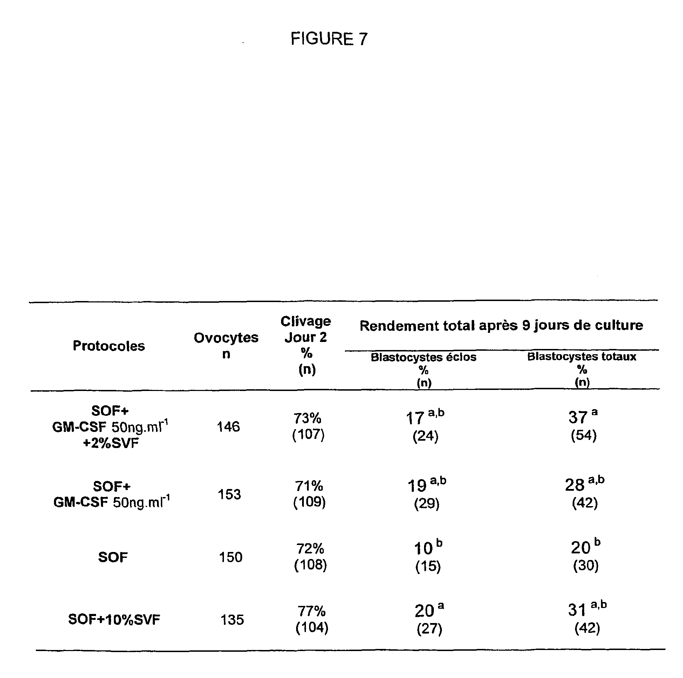

De la même manière, le tableau représenté à la figure 7 montre l'incidence du GM-CSF recombinant en présence ou en absence de SVF (sérum de veau foetal), sur le développement d'embryons produits in vitro et cultivés jusqu'au neuvième jour après la fécondation.

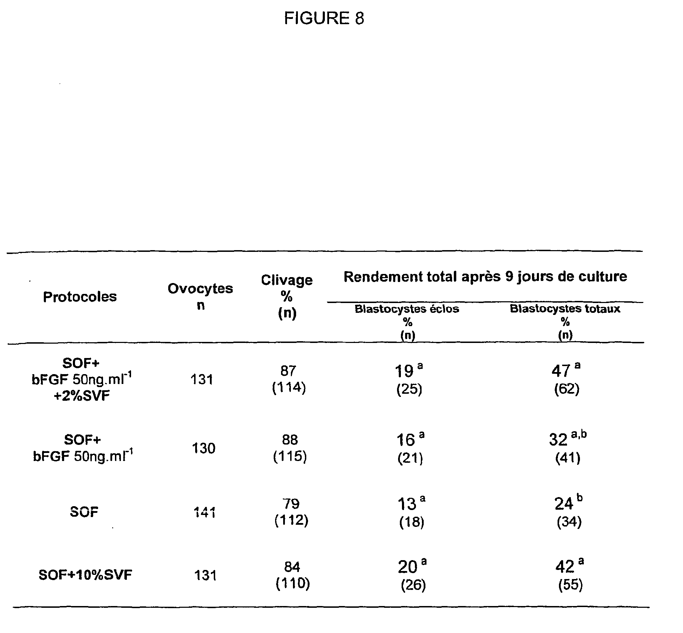

Le tableau représenté à la figure 8 illustre quant à lui l'incidence du bFGF recombinant en présence ou en absence de SVF (sérum de veau foetal), sur le développement d'embryons produits in vitro et cultivés jusqu'au neuvième jour après la fécondation.

Rien ne prouvait à partir des résultats obtenus dans les figures 4 à 8, qu'une combinaison de ces facteurs de croissance ou cytokines pouvait fournir les résultats montrés aux figures 1 à 3. Par ailleurs, l'état de la technique ne suggérait pas les résultats des effets des GM-CSF et bFGF (sans SVF) des figures 7 et 8 ni l'ampleur des résultats obtenus avec le TGFβ1, l'IGF-I et le LIF exempts de SVF des figures 4, 5, 6.

## Revendications

1. Milieu synthétique de culture et/ou de congélation et/ou de conservation et/ou de transfert d'embryons ou de cellules d'origine humaine ou animale, ce milieu se présentant sous forme d'une solution comprenant un milieu de base exempt de protéines naturelles d'origine animale ou humaine, les constituants de base de ce milieu de base incluant des sels essentiellement de calcium, de sodium et de potassium, au moins un acide aminé ou un dérivé d'acide aminé, de préférence des vitamines, et éventuellement au moins un antibiotique,
**caractérisé en ce que** ledit milieu contient, en sus du milieu de base, au moins un succédané aux protéines naturelles d'origine animale ou humaine, ce succédané à base de molécules recombinantes comprenant au moins la combinaison du TGF β1 (Transforming growth factor β1) et de l'IGF-I (Insulin-like growth factor I), avec au moins un facteur de croissance ou cytokine choisi dans le groupe constitué par le LIF (Leukemia inhibitory factor), le GM-CSF (Granulocyte macrophage colony stimulating factor) et le bFGF (fibroblast growth factor basique).

2. Milieu synthétique de culture et/ou de congélation et/ou de conservation et/ou de transfert d'embryons ou de cellules selon la revendication 1,
**caractérisé en ce que** le succédané comprend au moins la combinaison du TGF β1 et de l'IGF-I avec au moins deux facteurs de croissance ou cytokines choisis dans le groupe constitué par le LIF, le GM-CSF et le bFGF.

3. Milieu synthétique de culture et/ou de congélation et/ou de conservation et/ou de transfert d'embryons ou de cellules selon la revendication 1,
**caractérisé en ce que** le succédané aux protéines naturelles d'origine animale ou humaine comprend au moins la combinaison du TGF β1, de l'IGF-I et du LIF.

4. Milieu synthétique de culture et/ou de congélation et/ou de conservation et/ou de transfert d'embryons ou de cellules selon la revendication 1,
**caractérisé en ce que** le succédané aux protéines naturelles d'origine animale ou humaine comprend au moins la combinaison du TGF β1, de l'IGF-I, du LIF, du GM-CSF, du bFGF et de l'IGF-II.

5. Milieu de culture selon l'une des revendications 1 à 4,
**caractérisé en ce que** les facteurs de croissance ou cytokines sont présents chacun dans le milieu à une concentration comprise dans la plage [10 - 100] ng/mL, de préférence voisine de 50 ng/mL.

6. Milieu de culture selon l'une des revendications 1 à 5,
**caractérisé en ce que** le milieu de base est obtenu par mélange d'au moins cinq solutions, la première solution présentant, pour un litre de solution aqueuse, la composition suivante :
| | |
|---|---|
| NaCl (g) | 40 - 80 g/L |
| KH₂PO₄ (g) | 0,5 - 2,5 g/L |
| KCl | 4 - 6 g/L |
| MgSO₄7H₂O | 1 - 3 g/L |
| Na - Lactate (mL) | 5 - 7 mL/L |
| H₂O | |
la deuxième solution présentant, pour un litre de solution aqueuse, la composition suivante :
| | |
|---|---|
| NaHCO₃(g) | 15 - 30 g/L |
| Rouge de phénol | 0,05 - 0,2 g/L |
la troisième solution présentant pour un litre de solution aqueuse, la composition suivante :
| | |
|---|---|
| Na - pyruvate (g) | 6 - 10 g/L |
la quatrième solution présentant pour un litre de solution aqueuse, la composition suivante :
| | |
|---|---|
| CaCl₂2H₂O(g) | 20 - 30 g/L |
la cinquième solution, correspondant au milieu de base, présentant la composition telle que suit :
| | |
|---|---|
| H₂O (g) | |
| Citrate de sodium tribasique (g) | 0,05 - 0,2 g/L |
| Myo-inositol (g) | 0,4 - 0,6 g/L |
| Première solution (mL) | 10 % en volume |
| Deuxième solution (mL) | 10 % en volume |
| Troisième solution (mL) | 1 % en volume |
| Quatrième solution (mL) | 1 % en volume |
| BME 50x (mL) | 20 - 40 mL/L |
| MEM 100x (mL) | 5 - 20 mL/L |
| Glutamine 200 mM (mL) | 0,5 - 2 mL/L |
| Gentamycine (mL) | 4 - 6 mUL |
le milieu BME ayant une composition exprimée en g/L telle que suit :
| | |
|---|---|
| L-Arginine-HCl | 1,05 g/L |
| L-Cystine | 0,6 g/L |
| L-Histidine | 0,4 g/L |
| L-Isoleucine | 1,3 g/L |
| L-Leucine | 1,3 g/L |
| L-Lysine-HCl | 1,849 g/L |
| L-Methionine | 0,375 g/L |
| L-Phénylalanine | 0,825 g/L |
| L-Threonine | 1,2 g/L |
| L-Tryptophane | 0,2 g/L |
| L-Tyrosine | 0,9 g/L |
| L-Valine | 1,175 g/L |
tandis que le milieu MEM 100x a une composition exprimée en g/L telle que suit :
| | |
|---|---|
| L-Alanine | 0,89 g/L |
| L-Asparagine-H₂O | 1,5 g/L |
| Acide L-Aspartique | 1,33 g/L |
| Acide L-Glutamique | 1,47 g/L |
| Glycine | 0,75 g/L |
| L-Proline | 1,15 g/L |
| L-Sérine | 1,05 g/L |

7. Milieu de culture selon l'une des revendications 1 à 6,
**caractérisé en ce que** le milieu de base comprend en outre au moins un agent cryoprotecteur choisi de préférence dans le groupe constitué par l'éthylène glycol 0,15 M, le glycérol 0,1 M et le propylène glycol 0,15 M.

8. Utilisation de la combinaison du TGF β1 recombinant, de l'IGF-I recombinant et d'au moins un facteur de croissance ou cytokine recombinant choisi dans le groupe constitué par le LIF, le GM-CSF et le bFGF en tant que substitut de protéines naturelles d'origine animale ou humaines telles que le sérum de veau foetal dans la fabrication de milieu de culture et/ou de congélation et/ou de conservation et/ou de transfert d'embryons ou de cellules, d'origine humaine ou animale.

## Claims

1. Synthetic medium for the culture and/or freezing and/or preservation and/or transfer of embryos or cells of human or animal origin, this medium being in the form of a solution comprising a base medium free from natural proteins of animal or human origin, the basic constituents of this base medium including salts essentially of calcium, sodium and potassium, at least one amino acid or an amino acid derivative, preferably vitamins, and possibly at least one antibiotic,
**characterised in that** said medium contains, in addition to the base medium, at least one substitute for natural proteins of animal or human origin, this substitute based on recombinant molecules comprising at least the combination of TGF β1 (Transforming Growth Factor β1) and IGF-I (Insulin-like Growth Factor I), with at least one growth factor or cytokine chosen from the group consisting of LIF (Leukaemia Inhibitory Factor), GM-CSF (Granulocyte Macrophage Colony Stimulating Factor) and bFGF (basic Fibroblast Growth Factor).

2. Synthetic medium for the culture and/or freezing and/or preservation and/or transfer of embryos or cells according to Claim 1,
**characterised in that** the substitute comprises at least the combination of TGF β1 and IGF-I with at least two growth factors or cytokines chosen from the group consisting of LIF, GM-CSF and bFGF.

3. Synthetic medium for the culture and/or freezing and/or preservation and/or transfer of embryos or cells according to Claim 1,
**characterised in that** the substitute for natural proteins of animal or human origin comprises at least the combination of TGF β1, IGF-I and LIF.

4. Synthetic medium for the culture and/or freezing and/or preservation and/or transfer of embryos or cells according to Claim 1,
**characterised in that** the substitute for natural proteins of animal or human origin comprises at least the combination of TGF β1, IGF-I, LIF, GM-CSF, bFGF and IGF-II.

5. Culture medium according to one of Claims 1 to 4,
**characterised in that** the growth factors or cytokines are each present in the medium at a concentration lying in the range [10 - 100] ng/ml, preferably close to 50 ng/ml.

6. Culture medium according to one of Claims 1 to 5,
**characterised in that** the base medium is obtained by mixing at least five solutions, the first solution having, for one litre of aqueous solution, the following composition:
| | |
|---|---|
| NaCl (g) | 40 - 80 g/l |
| KH₂PO₄ (g) | 0.5 - 2.5 g/l |
| KCl | 4 - 6 g/l |
| MgSO₄7H₂O | 1 - 3 g/l |
| Na-lactate (ml) | 5 - 7 ml/l |
| H₂O | |
the second solution having, for one litre of aqueous solution, the following composition:
| | |
|---|---|
| NaHCO₃ (g) | 15 - 30 g/l |
| Phenol red | 0.05 - 0.2 g/l |
the third solution having, for one litre of aqueous solution, the following composition:
| | |
|---|---|
| Na-pyruvate (g) | 6 - 10 g/l |
the fourth solution having, for one litre of aqueous solution, the following composition:
| | |
|---|---|
| CaCl₂2H₂O (g) | 20 - 30 g/l |
the fifth solution, corresponding to the base medium, having the composition as follows:
| | |
|---|---|
| H₂O (g) | |
| Tribasic sodium citrate (g) | 0.05 - 0.2 g/l |
| Myo-inositol (g) | 0.4 - 0.6 g/l |
| First solution (ml) | 10% by volume |
| Second solution (ml) | 10% by volume |
| Third solution (ml) | 1% by volume |
| Fourth solution (ml) | 1% by volume |
| BME 50x (ml) | 20 - 40 ml/l |
| MEM 100x (ml) | 5 - 20 ml/l |
| Glutamine 200 mM (ml) | 0.5 - 2 ml/l |
| Gentamicin (ml) | 4 - 6 ml/l |
the BME medium having a composition expressed in g/l as follows:
| | |
|---|---|
| L-Arginine-HCl | 1.05 g/l |
| L-Cystine | 0.6 g/l |
| L-Histidine | 0.4 g/l |
| L-Isoleucine | 1.3 g/l |
| L-Leucine | 1.3 g/l |
| L-Lysine-HCl | 1.849 g/l |
| L-Methionine | 0.375 g/l |
| L-Phenylalanine | 0.825 g/l |
| L-Threonine | 1.2 g/l |
| L-Tryptophan | 0.2 g/l |
| L-Tyrosine | 0.9 g/l |
| L-Valine | 1.175 g/l |
whilst the MEM 100x medium has a composition expressed in g/l as follows:
| | |
|---|---|
| L-Alanine | 0.89 g/l |
| L-Asparagine-H₂O | 1.5 g/l |
| L-Aspartic acid | 1.33 g/l |
| L-Glutamic acid | 1.47 g/l |
| Glycine | 0.75 g/l |
| L-Proline | 1.15 g/l |
| L-Serine | 1.05 g/l |

7. Culture medium according to one of Claims 1 to 6,
**characterised in that** the base medium furthermore comprises at least one cryoprotective agent preferably chosen from the group consisting of 0.15 M ethylene glycol, 0.1 M glycerol and 0.15 M propylene glycol.

8. Use of the combination of recombinant TGF β1, recombinant IGF-I and at least one recombinant growth factor or cytokine chosen from the group consisting of LIF, GM-CSF and bFGF as a substitute for natural proteins of animal or human origin such as foetal calf serum in the production of a medium for the culture and/or freezing and/or preservation and/or transfer of embryos or cells, of human or animal origin.

## Patentansprüche

1. Synthetisches Medium zur Züchtung und/oder zum Einfrieren und/oder zur Aufbewahrung und/oder zur Übertragung menschlicher oder tierischer Embryonen oder Zellen, das in Form einer Lösung vorliegt, die ein Grundmedium umfasst, das frei von natürlichen tierischen oder menschlichen Proteinen ist, wobei die Grundbestandteile des Mediums Salze, im Wesentlichen Calcium-, Natrium- und Kaliumsalze, mindestens eine Aminosäure oder ein Aminosäurederivat, vorzugsweise Vitamine und gegebenenfalls mindestens ein Antibiotikum sind, **dadurch gekennzeichnet, dass** das Medium außer dem Grundmedium mindestens einen Ersatzstoff für natürliche tierische oder menschliche Proteine enthält, wobei der rekombinante Moleküle enthaltende Ersatzstoff mindestens die Kombination aus dem TGF-β1 (transformierender Wachstumsfaktor β1) und dem IGF-I (insulinähnlicher Wachstumsfaktor I) mit mindestens einem Wachstumsfaktor oder Zytokin aus der Gruppe, die von dem LIF (Leukämie inhibierender Faktor), dem GM-CSF (Granulozyten-Makrophagen-Kolonie stimulierender Faktor) und dem bFGF (basischer Fibroblasten-Wachstumsfaktor) gebildet wird, umfasst.

2. Synthetisches Medium zur Züchtung und/oder zum Einfrieren und/oder zur Aufbewahrung und/oder zur Übertragung von Embryonen oder Zellen nach Anspruch 1, **dadurch gekennzeichnet, dass** der Ersatzstoff mindestens die Kombination aus dem TGF-β1 und dem IGF-I mit mindestens zwei Wachstumsfaktoren oder Zytokinen aus der Gruppe, die von dem LIF, dem GM-CSF und dem bFGF gebildet wird, umfasst.

3. Synthetisches Medium zur Züchtung und/oder zum Einfrieren und/oder zur Aufbewahrung und/oder zur Übertragung von Embryonen oder Zellen nach Anspruch 1, **dadurch gekennzeichnet, dass** der Ersatzstoff für natürliche tierische oder menschliche Proteine mindestens die Kombination aus dem TGF-β1, dem IGF-I und dem LIF umfasst.

4. Synthetisches Medium zur Züchtung und/oder zum Einfrieren und/oder zur Aufbewahrung und/oder zur Übertragung von Embryonen oder Zellen nach Anspruch 1, **dadurch gekennzeichnet, dass** der Ersatzstoff für natürliche tierische oder menschliche Proteine mindestens die Kombination aus TGF-β1, dem IGF-I, dem LIF, dem GM-CSF, dem bFGF und dem IGF-II umfasst.

5. Zuchtmedium nach einem der Ansprüche 1 bis 4, **dadurch gekennzeichnet, dass** die Wachstumsfaktoren oder Zytokine in dem Medium jeweils in einer Konzentration in einem Bereich von [10 - 100] ng/ml, vorzugsweise etwa 50 ng/ml, enthalten sind.

6. Zuchtmedium nach einem der Ansprüche 1 bis 5, **dadurch gekennzeichnet, dass** das Grundmedium durch Mischung von mindestens fünf Lösungen erhalten wird, wobei die erste Lösung in einem Liter wässriger Lösung die folgende Zusammensetzung aufweist:
| | |
|---|---|
| NaCl (g) | 40 - 80 g/l |
| KH₂PO₄ (g) | 0,5 - 2,5 g/l |
| KCl | 4 - 6 g/l |
| MgSO₄7H₂O | 1 - 3 g/l |
| Na-Lactat (ml) | 5 - 7 ml/l |
| H₂O | |
die zweite Lösung in einem Liter wässriger Lösung die folgende Zusammensetzung aufweist:
| | |
|---|---|
| NaHCO₃ (g) | 15 - 30 g/l |
| Phenolrot | 0,05 - 0,2 g/l |
die dritte Lösung in einem Liter wässriger Lösung die folgende Zusammensetzung aufweist:
| | |
|---|---|
| Na-Pyruvat (g) | 6 - 10 g/l |
die vierte Lösung in einem Liter wässriger Lösung die folgende Zusammensetzung aufweist:
| | |
|---|---|
| CaCl₂2H₂O (g) | 20 - 30 g/l |
die fünfte Lösung in einem Liter wässriger Lösung die folgende Zusammensetzung aufweist:
| | |
|---|---|
| H₂O (g) | |
| Trinatriumcitrat (g) | 0,05 - 0,2 g/l |
| Myo-Inositol (g) | 0,4 - 0,6 g/l |
| Erste Lösung (ml) | 10 Vol.-% |
| Zweite Lösung (ml) | 10 Vol.-% |
| Dritte Lösung (ml) | 1 Vol.-% |
| Vierte Lösung (ml) | 1 Vol.-% |
| BME 50x (ml) | 20 - 40 ml/l |
| MEM 100x (ml) | 5 - 20 ml/l |
| Glutamin 200 mM (ml) | 0,5 - 2 ml/l |
| Gentamycin (ml) | 4 - 6 ml/l |
wobei das Medium BME die folgende Zusammensetzung in g/l aufweist:
| | |
|---|---|
| L-Arginin-HCl | 1,05 g/l |
| L-Cystein | 0,6 g/l |
| L-Histidin | 0,4 g/l |
| L-Isoleucin | 1,3 g/l |
| L-Leucin | 1,3 g/l |
| L-Lysin HCl | 1,849 g/l |
| L-Methionin | 0,375 g/l |
| L-Phenylalanin | 0,825 g/l |
| L-Threonin | 1,2 g/l |
| L-Tryptophan | 0,2 g/l |
| L-Tyrosin | 0,9 g/l |
| L-Valin | 1,175 g/l |
während das Medium MEM 100x die folgende Zusammensetzung in g/l aufweist:
| | |
|---|---|
| L-Alanin | 0,89 g/l |
| L-Asparagin-H₂O | 1,5 g/l |
| L-Asparaginsäure | 1,33 g/l |
| L-Glutaminsäure | 1,47 g/l |
| Glycin | 0,75 g/l |
| L-Prolin | 1,15 g/l |
| L-Serin | 1,05 g/l |

7. Zuchtmedium nach einem der Ansprüche 1 bis 6, **dadurch gekennzeichnet, dass** das Grundmedium zudem mindestens ein Kälteschutzmittel umfasst, das vorzugsweise aus der von Ethylenglykol 0,15 M, Glycerin 0,1 M und Propylenglykol 0,15 M gebildeten Gruppe ausgewählt wird.

8. Verwendung der Kombination aus dem rekombinanten TGF-β1, dem rekombinanten IGF-I und mindestens einem rekombinanten Wachstumsfaktor oder Zytokin aus der Gruppe, die von dem LIF, dem GM-CSF und dem bFGF gebildet wird, als Ersatzstoff für natürliche tierische oder menschliche Proteine, wie fetales Kälberserum, bei der Herstellung eines Mediums zur Züchtung und/oder zum Einfrieren und/oder zur Aufbewahrung und/oder zur Übertragung menschlicher oder tierischer Embryonen oder Zellen.
